# EUROPEAN PATENT SPECIFICATION

(11) **EP 2 757 153 B1**
(45) Date of publication and mention of the grant of the patent: **03.07.2019**
(21) Application number: 12820708.1
(22) Date of filing: 02.08.2012
(51) Int. Cl.: C12N 15/09, C07K 14/47, C07K 16/18, C12Q 1/68, G01N 33/15, G01N 33/50, G01N 33/68

(54) **METHOD FOR ASSESSING MYELODYSPLASTIC SYNDROME OR MYELOID TUMOR PREDISPOSITION, POLYPEPTIDE AND ANTIBODY THEREFOR, AND CANDIDATE SCREENING METHOD FOR THERAPEUTIC DRUG OR PROPHYLACTIC DRUG THEREFOR**
VERFAHREN ZUR BEURTEILUNG DER PRÄDISPOSITION FÜR MYELODYSPLASTISCHES SYNDROM ODER MYELOISCHEN TUMOR, POLYPEPTID UND ANTIKÖRPER DAFÜR SOWIE KANDIDATENSCREENINGVERFAHREN FÜR THERAPEUTISCHEN ODER PROPHYLAKTISCHEN WIRKSTOFF
MÉTHODE D'ÉVALUATION DU SYNDROME MYÉLODYSPLASIQUE OU DE LA PRÉDISPOSITION À LA TUMEUR MYÉLOÏDE, POLYPEPTIDE ET ANTICORPS S'Y RAPPORTANT, ET PROCÉDÉ DE CRIBLAGE POUR IDENTIFIER DES CANDIDATS-MÉDICAMENTS DESTINÉS À LES TRAITER ET/OU À LES PRÉVENIR

(30) Priority: 02.08.2011 JP 2011169662
(43) Date of publication of application: 23.07.2014
(73) Proprietor: The University of Tokyo, Bunkyo-ku, Tokyo 113-8654 (JP)
(72) Inventor: OGAWA, Seishi, Tokyo 113-8654 (JP); SANADA, Masashi, Tokyo 113-8654 (JP); YOSHIDA, Kenichi, Kyoto 606-8501 (JP)
(74) Representative: Stüven, Ralf
(86) International application number: PCT/JP2012/069711
(87) International publication number: WO 2013/018866

(56) References cited:
- WO-A1-2009/150229
- WO-A2-2006/034278
- WO-A2-2011/143656
- JP-A- 2005 295 948
- MULLIGHAN CHARLES G: "TET2 mutations in myelodysplasia and myeloid malignancies.", NATURE GENETICS JUL 2009, vol. 41, no. 7, July 2009 (2009-07), pages 766-767, XP002740465, ISSN: 1546-1718
- BEJAR RAFAEL ET AL: "Unraveling the molecular pathophysiology of myelodysplastic syndromes", JOURNAL OF CLINICAL ONCOLOGY, LIPPINCOTT WILLIAMS & WILKINS, USA, vol. 29, no. 5, 10 February 2011 (2011-02-10), pages 504-515, XP009162755, ISSN: 1527-7755
- SANADA M ET AL: "Gain-of-function of mutated C-CBL tumour suppressor in myeloid neoplasms", NATURE 20090813 NATURE PUBLISHING GROUP GBR, vol. 460, no. 7257, 13 August 2009 (2009-08-13), pages 904-908, XP055193588, ISSN: 0028-0836
- DOGAN Y ET AL: "Quantification of transforming capacity and cooperation of defined genetic alterations in myeloid malignancies", EXPERIMENTAL HEMATOLOGY, ELSEVIER INC, US, vol. 38, no. 1, 1 January 2010 (2010-01-01), pages 11-19, XP026799293, ISSN: 0301-472X, DOI: 10.1016/J.EXPHEM.2009.10.006 [retrieved on 2009-11-11]
- KOHLMANN A. ET AL.: 'Next-generation sequencing technology reveals a characteristic pattern of molecular mutations in 72.8% of chronic myelomonocytic leukemia by detecting frequent alterations in TET2, CBL, RAS, and RUNX1' J. CLIN. ONCOL. vol. 28, no. 24, 2010, pages 3858 - 3865, XP055142758
- SEIJI OGAWA: 'Jisedai Sequence Gijutsu o Katsuyo shita MDS no Shinki Gen'in Idenshi no Dotei' FUOSEI HINKETSU NO CHIYURITSU KOJO O MEZASHITA BUNSHI - MEN'EKI BYOTAI KENKYU HEISEI 22 NENDO SOKATSU - BUNTAN KENKYU HOKOKUSHO March 2011, pages 13 - 14, XP008173302
- BEJAR R. ET AL.: 'Unraveling the molecular pathophysiology of myelodysplastic syndromes' J. CLIN. ONCOL. vol. 29, no. 5, January 2011, pages 504 - 515, XP009162755
- YOSHIDA K. ET AL.: 'Frequent pathway mutations of splicing machinery in myelodysplasia' NAURE vol. 478, no. 7367, September 2011, pages 64 - 69, XP055142763
- GRAUBERT TA. ET AL.: 'Recurrent mutations in the U2AF1 splicing factor in myelodysplastic syndromes' NAT. GENET. vol. 44, no. 1, December 2011, pages 53 - 57, XP055142764
- THOL F. ET AL.: 'Frequency and prognostic impact of mutations in SRSF2, U2AF1, and ZRSR2 in patients with myelodysplastic syndromes' BLOOD vol. 119, no. 15, March 2012, pages 3578 - 3584, XP055066386

## Description

### TECHNICAL FILED

The present invention relates to a method of evaluating predisposition for myelodysplastic syndromes or a myelogenous tumor, and a polypeptide and antibody therefor as well as a method of screening for a candidate therapeutic agent or a candidate prophylactic agent for myelodysplastic syndromes or a myelogenous tumor.

### BACKGROUND ART

Myelodysplastic syndromes (MDS), which is a disorder where erythrocytes, leucocytes and platelets are decreased in peripheral blood although hematopoietic cells are produced in bone marrow, are thought to be caused by oncogenic transformation at the stem cell level because it often progresses to acute leukemia. Currently, the syndrome is classified as refractory anemia (RA), refractory anemia with ringed sideroblasts (RARS), refractory cytopenia with multikineage dysplasia (RCMD), refractory anemia with multikineage dysplasia with ringed sideroblasts (RCMD-RS), refractory anemia with excess blasts (RAEB), 5q-syndrome and the like. In particular, acute myelogenous leukemia (AML) is the most important complication, but a process in which myelodysplastic syndromes progresses to leukemia has not been known.

A myelogenous tumor, which is characterized in that it may progress to uncontrolled production of dysplastic blood cells and acute myelogenous leukemia, is also an associated disorder of myelodysplastic syndromes.

In order to prevent the progress of myelodysplastic syndromes or a myelogenous tumor to acute myelogenous leukemia, early diagnosis and early treatment are required. However, conveniently, only a blood test which may be used only for confirmed diagnosis of the disorders has been available.

Since a clinical course is indolent before leukemic transformation and ineffective hematopoiesis which are symptoms of myelodysplastic syndromes occur, an onset different from that involved in primary acute myelogenous leukemia is suggested. Nonetheless, the genetic basis of these disorders has not fully elucidated. Some gene mutations and cytogenetic changes are involved in the onset, and *RAS, TP53, RANX1, ASXL1, c-CBL, IDH1*/*2, TET2* and *EZH2* are included in the gene targets in which a mutation is most frequently found in myeloid dysplasia. However, for example, because known genetic alterations are not found in about 20% of the cases, a mutation in this set of the genes can not fully explain the onset. Interesting insights for this problem have been provided from the research results about haploinsufficiency in *RPS14* and *miR-145*/*146* associated with 5q-syndrome. However, in particular, a genetic alteration causing a metaplasia phenotype thereof has not been sufficiently understood.

Meanwhile, the recent development of massively parallel sequencing technology has provided a new opportunity for probing a genetic alteration across the entire genome or the entire protein coding sequences in human cancer at one nucleotide level.

### CITATION LIST

### Nonpatent Literatures

Nonpatent Literature 1: Wahl, M.C., Will, C. L. & Luhrmann, R. The spliceosome design principles of a dynamic RNP machine. Cell 136, 701-718 (2009)
Nonpatent Literature 2: Tronchere, H., Wang, J. & Fu, X. D. A proteinrelatedto splicing factor U2AF35 that interests with U2AF65 and SR proteins insplicing of pre-mRNA. Nature 388, 397-400(1997)
Nonpatent Literature 3: Calvo, S. E., et al. High-throughput, pooled sequencingidentifies mutations in NUBPL and FOXRED1 in human complex I deficiency. NatGenet 42, 851-858 (2010)
Nonpatent Literature 4: Bevilacqua, L., et al. Apopulation-specific HTR2B stop codon predisposes to severe impulsivity. Nature 468, 1061-1066 (2010)
Nonpatent Literature 5: Chen. M. & Manley, J. L. Mechanisms of alternative splicing regulation insights frommolecular and genomics approaches. Nat Rev Mol Cell Biol 10, 741-754(2009)
Nonpatent Literature 6: Subramanian, A., et al. Gene set enrichment analysis a knowledge-based approach for interpretinggenome-wide expression profiles. Proc Natl Acad Sci USA 102, 15545-15550 (2005)
Nonpatent Literature 7: Bhuvanagiri, M., Schlitter, A. M, Hentze, M. W. & Kulozik, A. E. NMD RNA biology meets human geneticmedicine. Biochem J430, 365-377 (2010)
Nonpatent Literature 8: Maquat, L. E. Nonsense-mediatedmRNA decay splicing, translation and mRNP dynamics. NatRev Mol Cell Biol 5, 89-99 (2004)
Nonpatent Literature 9: Shen, H., Zheng, X., Luecke, S. & Green, M.R. The U2AF35-related protein Urpcontacts the 3'splice site to promote U12-type intron splicing and the secondstep of U2-type intron spliclng. Genes Dev24, 2389-2394 (2010)

### SUMMARY OF INVENTION

### TECHNICAL PROBLEM

Accordingly, an object of the present invention is to provide, based on genetic diagnosis using the massively parallel sequencing technology, a method of evaluating predisposition for myelodysplastic syndromes or a myelogenous tumor, and a polypeptide and antibody therefor as well as a method of screening for a candidate therapeutic agent or a candidate prophylactic agent for myelodysplastic syndromes or a myelogenous tumor.

### SOLUTION TO PROBLEM

The method of evaluating predisposition for myelodysplastic syndromes and a myelogenous tumor according to the present invention is a method of evaluating whether a subject has predisposition for possible development of myelodysplastic syndromes or a myelogenous tumor, the method comprising the step of detecting a gene mutation in at least one gene of the *U2AF35* gene (also referred to as *U2AF1*)*,* the *ZRSR2* gene, the *SFRS2* gene and the *SF3B1* gene using a sample containing human genes of the subject.

Here, in a case where at least one of the following mutations: a substitution of S with F or Y at an amino acid residue at position 34 of a protein translated from the *U2AF35* gene, a substitution of Q with R or P at an amino acid residue at position 157 of the protein translated from the *U2AF35* gene, any inactivating mutation in a protein translated from the *ZRSR2* gene, a substitution of P with H or L or R at an amino acid residue at position 95 of a protein translated from the *SFRS2* gene, a substitution of K with E at an amino acid residue at position 700, a substitution of E with D at an amino acid residue at position 622, a substitution of H with Q or D at an amino acid residue at position 662, a substitution of K with N or T or E or R at an amino acid residue at position 666 of a protein translated from the *SF3B1* gene are detected, a subject is evaluated as having predisposition for possible development of myelodysplastic syndromes or a myelogenous tumor, and conversely, in a case where not detected, the subject may be indirectly evaluated as not likely having the predisposition.

In order to detect an amino acid substitution described above, a mutation in a gene corresponding to the amino acid substitution may be detected, or a mutation in a protein and a polypeptide translated therefrom may be detected.

Note that as amino acid abbreviations, S represents Ser (serine), F represents Phe (phenylalanine), Y represents Tyr (tyrosine), Q represents Gln (glutamine), R represents Arg (arginine), P represents Pro (proline), E represents Glu (glutamic acid), X represents Xaa (unknown or other amino acids), H represents His (histidine), L represents Leu (leucine), R represents Arg (arginine), K represents Lys (lysine), D represents Asp (aspartic acid), N represents Asn (asparagine) and T represents Thr (threonine).

A polypeptide according to the present invention comprises at least a portion of the *U2AF35* gene, and has at least one of the substitution of S with F or Y at an amino acid residue at position 34 or the substitution of Q with R or P at an amino acid residue at position 157, the polypeptide being able to serve as a marker for evaluating predisposition for myelodysplastic syndromes or a myelogenous tumor.

Similarly, a polypeptide according to the present invention may comprise at least a portion of the *ZRSR2* gene, and has any inactivating amino acid mutation, the polypeptide being able to serve as a marker for evaluating predisposition for myelodysplastic syndromes or a myelogenous tumor.

A polypeptide according to the present invention may comprise at least a portion of the *SFRS2* gene, and has at least one of the substitutions of P with H or L or R at an amino acid residue at position 95, the polypeptide being able to serve as a marker for evaluating predisposition for myelodysplastic syndromes or a myelogenous tumor.

A polypeptide according to the present invention may comprise at least a portion of the *SF3B1* gene, and has at least one of the substitution of K with E at an amino acid residue at position 700, the substitution of E with D at an amino acid residue at position 622, the substitution of H with Q or D at an amino acid residue at position 662, the substitution of K with N or T or E or R at an amino acid residue at position 666, the polypeptide being able to serve as a marker for evaluating predisposition for myelodysplastic syndromes or a myelogenous tumor.

A polypeptide according to the present invention may comprise an amino acid sequence in which one or several amino acids are deleted, substituted or added in any of the above polypeptides, the polypeptide functioning as an antigen against an antibody, the antibody recognizing one of the original polypeptides.

An antibody according to the present invention is characterized by recognizing one of the above polypeptides.

The method of screening for a candidate therapeutic agent or a candidate prophylactic agent for myelodysplastic syndromes and a myelogenous tumor according to the present invention is a method of screening for a pharmaceutical agent for myelodysplastic syndromes or a myelogenous tumor, the method comprising the steps of evaluating whether a test substance can inhibit an expression or an activity of a protein translated from at least one gene of the *U2AF35* gene, the *ZRSR2* gene, the *SFRS2* gene and the *SF3B1* gene using a sample containing human genes of a subject, and selecting the test substance capable of inhibiting the expression or the activity of the protein translated from said at least one gene as an effective substance for preventing or treating a state or a disease resulted from myelodysplastic syndromes or a myelogenous tumor.

### ADVANTAGEOUS EFFECTS OF INVENTION

The present invention can provide simple and accurate diagnosis for myelodysplastic syndromes and a myelogenous tumor by using genetic diagnosis, contributing to early treatment and prevention. Further, the present invention can also be useful for pathology classification and therapy selection.

### BRIEF DESCRIPTION OF DRAWINGS

Fig. 1 shows a table indicating gene mutations which repeatedly appear in 32 cases revealed from the whole exon analysis.
Fig. 2 shows (a) a diagram illustrating gene mutations frequency found in the splicing complexes E/A and components thereof, (b) diagrams illustrating mutations in multiple components of the splicing complexes E/A in a myeloid tumor.
Fig. 3 shows graphs indicating (a) frequencies of spliceosome pathway genes, (b) distributions thereof.
Fig. 4 shows (a) a photograph of western blot analysis in which a S34F mutant of *U2AF35* was expressed, (b) a graph showing the activation of the NMD pathway thereof, (c) a graph showing the results of qPCR thereof, (d) a graph showing an expression ratio of exons and introns thereof, (e) a graph showing an expression ratio of exons and introns thereof.
Fig. 5 shows (a) a photograph of western blot analysis in which doxycycline inducibility of a mutant and the wild-type *U2AF35* was expressed, (b) graphs showing changes in the cell cycle thereof, (c) a graph showing changes in the cell cycles thereof, (d) a graph showing induction of apoptosis thereof, (e) a graph showing functional analysis of a mutant *U2AF35* thereof.

### DESCRIPTION OF EMBODIMENTS

Below, embodiments according to the present invention will be described. Prior art documents and conventionally known technologies can be appropriately used in aid of changing designs of the embodiments.

The present inventors have conducted the somatic cell exome analysis using blood samples from 32 cases of myelodysplastic syndromes patients and healthy persons, and have obtained findings that there are abnormalities in the genes involved in RNA splicing and that amino acid substitutions occur therein. Because these gene abnormalities are found in 40% of the patient samples, testing for the gene abnormalities can identify whether a patient has a myeloid abnormality. Further, as a result of investigating 159 cases of myelodysplastic syndromes patients, 88 cases of chronic myelomonocyte leukemia (CMML) patients and the like, similar abnormalities have been found in about 40% of them. Further, this is the first case which can prove that RNA splicing abnormality may trigger an onset of a disease.

The present inventors have identified mutations in the *U2AF35* gene, the *ZRSR2* gene, the *SFRS2* gene and the *SF3B1* gene found in patients of myelodysplastic syndromes or a myelogenous tumor.

Specifically, they are a substitution of S with F or Y at an amino acid residue at position 34 of a protein translated from the *U2AF35* gene, a substitution of Q with R or P at an amino acid residue at position 157 of the protein translated from the *U2AF35* gene, any inactivating mutation in a protein translated from the *ZRSR2* gene, a substitution of P with H or L or R at an amino acid residue at position 95 of a protein translated from the *SFRS2* gene, a substitution of K with E at an amino acid residue at position 700, a substitution of E with D at an amino acid residue at position 622, a substitution of H with Q or D at an amino acid residue at position 662, a substitution of K with N or T or E or R at an amino acid residue at position 666 of a protein translated from the *SF3B1* gene. Note that for the mutations in the *ZRSR2* gene, the substitutions of E with X at an amino acid residue at position 362 and the like are mentioned in Example, but they include any inactivation mutation in a protein translated.

In a case where any of these is detected, a subject is evaluated as having predisposition for possible development of myelodysplastic syndromes or a myelogenous tumor, and conversely, in a case where not detected, the subject may be indirectly evaluated as not likely having the predisposition.

A polypeptide having any of the above gene mutations can be used as a marker for evaluating predisposition for myelodysplastic syndromes or a myelogenous tumor.

Note that a polypeptide refers to a substance in which two or more amino acid residues are connected via a peptide bond, including relatively short peptides or so-called oligopeptides and long chains called proteins. When producing an antibody for detecting a polypeptide, the polypeptide may comprise an amino acid residue other than the genetically encoded 20 amino acid residues or a modified amino acid residue. Modifications therein include acetylation, acylation, ADP-ribosylation, amidation, biotinylation, covalent bonding with a lipid and a lipid derivative, cross-linking formation, a disulfide bond, addition of a carbohydrate chain, addition of a GPI anchor, phosphorylation, prenylation and the like in a main chain having a peptide bond, an amino acid side chain, an amino terminus and a carboxyl terminus.

The polypeptide according to the present invention may further comprise one or several amino acid deletions, substitutions, additions in addition to the amino acid substitutions in the above gene products as long as it can serve as an antigen against an antibody which recognizes the polypeptide. These mutations may be included in a portion other than the epitope which is recognized by an antibody.

These polypeptides can be prepared by directly preparing the peptides by chemical synthesis. In addition, they can also be prepared by producing polynucleotides encoding these by the site directed mutagenesis method and the like, and then expressing them in an appropriate system.

These polypeptides can be prepared by isolating them from a sample such as blood cells taken from a patient of myelodysplastic syndromes or a myelogenous tumor in a case where the polypeptides are included as gene products of polynucleotides in the sample; by chemically synthesizing them based on the information of amino acid sequences including corresponding mutated amino acid residues; by transforming appropriate host cells by the recombinant DNA technology using appropriate polynucleotide-containing vectors and the like, and producing them in the transformed cells; and the like.

Further, a polynucleotide can be isolated by screening a cDNA library prepared from the total mRNA in a sample taken from a patient of myelodysplastic syndromes or a myelogenous tumor using a corresponding polynucleotide as a probe. Further, a polynucleotide can be prepared by introducing a base mutation into the cDNA of the wild type gene based on site directed mutagenesis using a commercially available mutation system and the like.

Such a polynucleotide may be amplified by the PCR (Polymerase Chain Reaction) method, the RT-PCR method, NASBA (Nucleic acid sequence based amplification) method, TMA (Transcription-mediated amplification) method, SDA (Strand Displacement Amplification) method and the like using, for example, genomic DNA or mRNA prepared from a sample such as blood cells obtained from a patient of myelodysplastic syndromes or a myelogenous tumor as a template, and using appropriate primers .

An antibody according to the present invention is characterized by specifically recognizing any of the above polypeptides. The antibody may be a polyclonal antibody or a monoclonal antibody. It is not limited to an antibody having the original intact structure, and may be various derivatives from the intact antibody such as fragments as long as a binding activity is maintained. These antibodies are useful as components for a detection system used for a method of detecting a mutated gene.

Antibody may be produced using a polypeptide or a complex of the polypeptide and a suitable adjuvant as an immunogen. For example, a polyclonal antibody can be obtained from blood serum after immunizing an animal with an immunogen.

A polypeptide can be detected, for example, by using immunologically specific reactions such as EIA and ELISA in which the antibody according to the present invention is used; by amino acid sequencing of a polypeptide such as a gas phase sequencer using the Edman method; by mass spectrometry such as the MALDI-TOF/MS method and the ESIQ-TOF/MS method.

### EXAMPLES

The whole exome sequencing of bone marrow-derived DNA was performed for 32 patients having myelodysplastic syndromes or a related myeloid tumor, using paired CD3 positive T cells or oral mucosa as a germline control. The whole exome approach is a well-established low cost and high performance method for obtaining a comprehensive registry of mutations in protein codes although non-coding mutations and gene rearrangements are undetectable. On average, 79% of the target sequences were analyzed with a depth of more than 20 folds. All candidates (N = 509) for a nonsynonymous single nucleotide mutation (SNV) and a small insertion/deletion (indel) were intensively checked by sequencing using the Sanger's method. Finally, 248 somatic mutations (7.8 per sample) including 191 missense mutations, 24 nonsense mutations, 9 splice site mutations and 24 frameshift induced indels were found. Together with a genome copy number profile obtained from the SNP array karyotype analysis, an overview of the myeloid dysplasia genome was obtained from this somatic mutation array.

An unbiased sample set of mutated genes including a majority of the gene targets frequently observed in myeloid dysplasia was used together with a typical genome copy number profile. As expected, mutations in the known gene targets accounted for only 13.3% of all detected mutations (N= 33), and the remaining 215 mutations were found in genes where a mutation had not been previously reported for these tumors. However, it was not easy to distinguish a driver event from a passenger event in the latter case. In many cases, a nonsense/frameshift mutation is likely to impair a protein function, and a target thereof will be a candidate for a tumor suppressor gene. Further, inclusion of common genetic pathways which have been implicated in myeloid dysplasia to date would support that they are not passenger events, but play a causative role.

Therefore, mutations in the TP53 related pathway genes *(DAPK1, TP53BP1)* and the genes involved in chromatin regulation *(PHF6, DOT1-L, PHF8)* were reasonable candidates for driver mutations. For example, there is a report that a Dot1-L deficient mouse developed severe anemia due to a defective erythrocyte production in the bone marrow. Congenital nonsense mutations in *PHF6* (Xq26.3) and *PMF8* (Xp11.2) are responsible for X-linked mental retardation syndrome. *PHF8* is a member of the histone demethylation enzyme family proteins which are widely accepted as mutation targets frequently observed in human cancers including myeloid dysplasia while loss-of-function mutations in *PHF6* have been reported in about 30% of T cell acute lymphoblastic leukemia (ALL).

Further, in the extended case series of Example, 11 mutations in *PHF6* were identified in 10 out of 164 cases of myeloid dysplasias. On the other hand, there was a possibility that mutations observed in multiple cases might be interesting driver mutations since they were likely to be frequently present in myeloid dysplasia. Indeed, 9 out of 12 genes in which mutations occurred frequently were included in the most frequent known target, and involved in a critically important gene pathway in myeloid dysplasia.

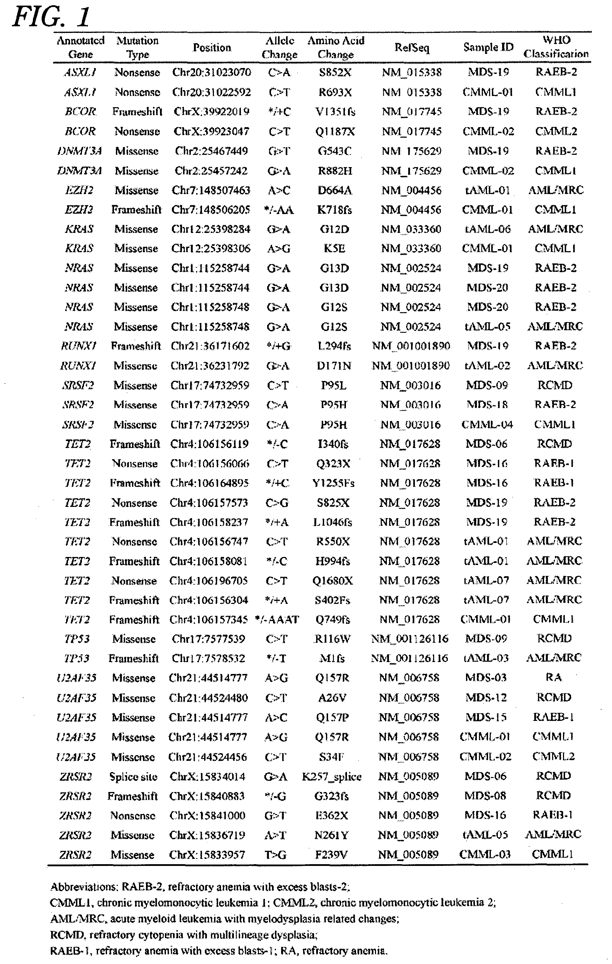

The remaining 3 genes (*URAF1, ZRSR2, SFRS2*) have not been reported to date, and have belonged to a common pathway known as the RNA splicing machinery, i.e., the spliceosome. Gene mutations which repeatedly appear in the 32 cases reveled by the whole exon analysis are shown in a table of Fig. 1.

Noticeably, when 3 genes (*SF3A1, SF3B1, PRPF40B*) which showed a mutation in a single case were further included, mutually exclusive mutations were found in 6 components of the splicing machinery in 15 out of 32 cases (47%).

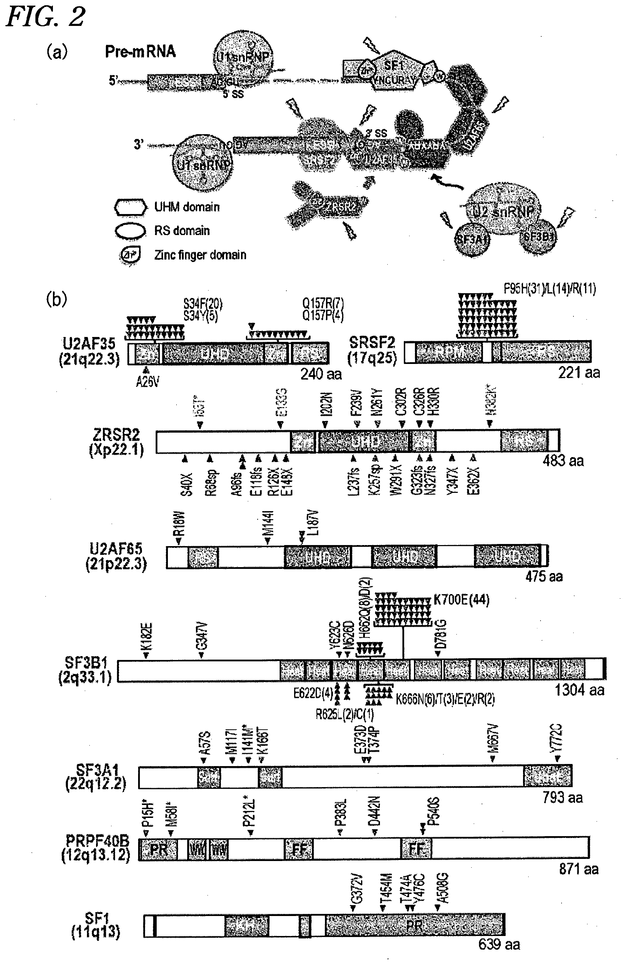

Fig. 2 (a) shows a diagram illustrating gene mutations frequency observed in the splicing complexes E/A and components thereof. Arrows indicate mutated components identified by the whole exome sequencing.

In the early stage of RNA splicing, at the same time when U1 snRNP is recruited to the 5'SS of Pre-mRNA during transcription, SF1 and a larger subunit U2AF2 of the U2 auxiliary factor (U2AF) bind to the branch point sequence (BPS) and the polypyrimidine region downstream thereof. A smaller subunit (U2AF35) of U2AF binds to the AG dinucleotide at 3'SS, and interacts with both U2AF2 and a SF protein (for example, SFRS2 (sc35)) through its UHD domain and SR domain, respectively, to form an immediate-early splicing complex (the E complex). ZRSR2, i.e., Urp also interacts with U2AF and a SR protein to play an essential role for RNA splicing. After 3'SS is recognized by these factors, U2 snRNP of the multi-component protein/snRNA complex comprising SF3A1 and SF3B1 is recruited to 3'SS to form the splicing complex A.

In myeloid dysplasia, multiple components of these splicing complexes are affected by gene mutations, resulting in novel and notable examples of pathway mutations in human cancers.

The majority of coding sequences (exons) of metazoan genes are separated by intervening non-coding sequences (introns) which needs to be removed from Pre-mRNA before mature mRNA is produced by a process called RNA splicing. Such functionality is achieved as follows: a set of small molecule nuclear ribonucleoprotein (snRNP) complexes (U1, U2, U4/5/6, U11/12) and many other protein components are recruited on Pre-mRNA in a controlled fashion, reorganized there and then released such that exon-intron boundaries are recognized. Subsequently, two transesterification reactions occur: first between a 5'-splicing site (5'SS) and the branch point sequence (BPS) and then between 5'SS and 3'SS such that the lariat intron is excised out to leave a connected exon.

A point which should be noted with regard to this is a fact that all mutated spliceosome components except for PRPF40B whose function in RNA splicing has not been elucidated are involved in the early stage of RNA splicing. SF1, and the U2 auxiliary factor (U2AF) comprising a U2AF2/U2AF35 heterodimer formed through physical interaction with a SR protein such as SFRS1 or SFRS2 (sc35) are involved in recognition of 3'SS and the adjacent polypyrimidine region. This is thought to be required to later recruit U2 snRNP comprising SF3A1 and SF3B1 to establish the splicing A complex (Nonpatent Literature 1) .

Meanwhile, ZRSR2 also known as Urp (an U2AF35 related protein) is another essential component of the splicing machinery. ZRSR2, which has a very similar structure as U2AF35, also physically interacts with U2AF2 as well SFRS1 and SFRS2, and plays a role which is different from that of its homolog U2AF35 (Nonpatent Literature 2).

In order to confirm and expand the initial findings from the whole exome sequencing, the above 6 genes in many myeloid tumors (N= 582), and additional 3 spliceosome related genes including *U2AF65, SF1,* and *SRSF1* were investigated for mutations by performing high throughput mutation screening of pooled DNA, and then confirming/identifying candidate mutations (Nonpatent Literatures 3 and 4).

As a result, total 219 mutations were identified in 209 out of 582 myeloid tumor samples by examining 313 tentative positive events from the pooled DNA screening. Mutations in four genes, *U2AF35* (N = 37), *SRSF2* (N = 56), *ZRSR2* (N = 23) and *SF3B1* (N = 79) accounted for most of the mutations, and mutation rates in *SF3A1* (N = 8), *PRPF40B* (N = 7), *U2AF65* (N = 4) and *SF1* (N = 5) were much lower.

Fig. 2 (b) shows diagrams illustrating mutations in multiple components of the splicing complexes E/A in myeloid tumors. Arrows indicate mutations in 6 spliceosome components of the splicing complexes E/A while squares indicate known domain structures.

Mutations in *U2AF35* and *SFRS2* (as well as *SF3B1* in some cases) are involved in different hot spots while mutations in *ZRSR2* are broadly distributed across its full length, and most of them are nonsense mutations which are responsible for early truncation of a protein or splice site mutations, i.e., indels.

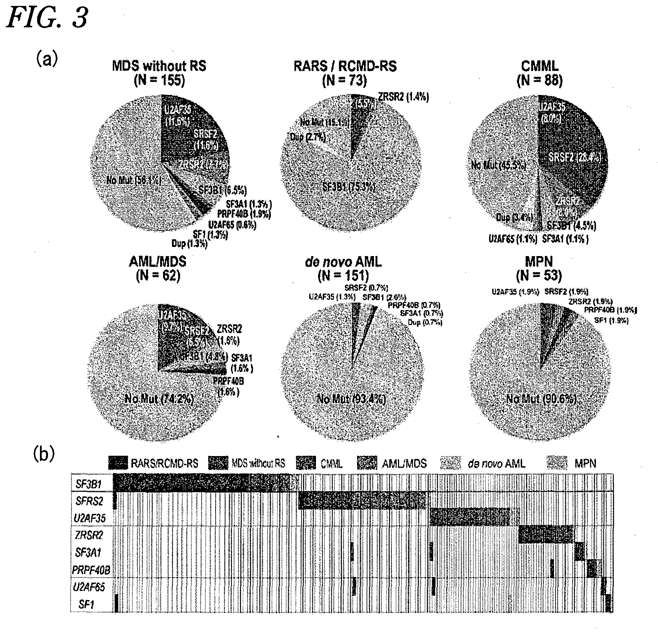

Fig. 3 (a) shows frequencies of spliceosome pathway mutations in 513 cases of myeloid tumors including the first 32 cases analyzed by the whole exome study. Frequencies are shown for each tumor type, i.e., MDS, CMML, AML/MDS (including both AML with myeloid dysplasia-related changes and treatment-related AML), primary AML and a myeloproliferative tumor (MPN).

Mutations in the splicing machinery were very specific for diseases showing characteristics of myeloid dysplasia such as MDS with increased ringed sideroblasts (84.9%) or MDS without increased ringed sideroblasts (43.9%), chronic myelomonocytic leukemia (CMML) (54.5%) and treatment-related AML or AML with myeloid dysplasia-related changes (25.8%), but were rare in primary AML (6.6%) and a myeloproliferative tumor (MPN) (9.4%). Further, surprisingly, for refractory anemia with ringed sideroblasts (RARS) and refractory anemia with multikineage dysplasia with ringed sideroblasts (RCMD-RS), many mutations were found in *SF3B1,* showing 82.6% and 76%, respectively.

Fig. 3 (b) shows distributions of two or more spliceosome pathway mutations for these eight genes. Diagnoses of patients are each shown in the top lane.

Mutually exclusive patterns of mutaions in these splicing pathway genes were observed in the large case series, suggesting that different gene mutations in splicing regulation result in a common result.

Meanwhile, frequencies of mutations showed a significant difference across disease types. Surprisingly, *SF3B1* mutations were found in the majority of the MDS cases characterized by increased ringed sideroblasts, i.e., refractory anemia with ringed sideroblasts (RARS) (19/23, i.e., 82.6%) and refractory anemia with multikineage dysplasia with ringed sideroblasts (≥15%) (RCMD-RS) (38/50, i.e., 76%) while the mutation frequency in other myeloid tumors was found to be much less. RARS and RCMD-RS account for 4.3% and 12.9% of the MDS cases, respectively. In these disorders, uncontrolled iron metabolism appears to be responsible for refractory anemia. Since the mutation frequency and specificity were very high as described above, *SF3B1* mutations were virtually pathognomonic for these MDS subtypes which are characterized by increased ringed sideroblasts, suggesting they are strongly involved in the onset of MDS of these categories. The frequency of *SRSF2* mutations was significantly higher in the CMML cases although it was not much striking.

Therefore, different mutations have a common effect on the E/A splicing complex while they may also have a different effect on cell functions, contributing to determining an individual disease phenotype. For example, it has been shown that *SRSF2* is also involved in regulation of DNA stability and that decrease in *SRSF2* may result in highly frequent DNA mutations. Interestingly in this regard, it was shown that a sample having a *SRSF2* mutation significantly had more mutations in other genes as compared with *U2AF35* mutations regardless of disease subtypes (p=0.001, multiple linear regression analysis).

Notably, suppose that A26V in one case is a rare exception, *U2AF35* mutations were mainly found at two highly conserved amino acid positions (S34 or Q157) within the zinc finger motifs at the N-terminus and the C-terminus adjacent to the UHM domain. *SRSF2* mutations were mainly found at P95 within an intervening sequence between the RPM domain and the RS domain. Similarly, *SF3B1* mutations were found to be mostly K700E, and to a lesser extent, were found at K666, H662 and E622, which are also conserved across species.

Although the frequently appearing amino acid locations found in these spliceosome genes strongly suggest that these mutations have a gain-of-function property, this is a scenario well proven even for other oncogene mutations including RAS mutations found at codons 12, 13 and 61 as well as V617F, the *JAK2* mutation, V600E, the *BRAF* mutation and recently found Y641, the *EZH2* mutation.

In contrast, 23 mutations in *ZRSR2* (Xp22.1) were widely distributed across the entire coding region (Fig. 2 (b)). Among these, 14 mutations were nonsense mutations or frameshift mutations, or were found in the splicing donor/acceptor sites, which may be responsible for early truncation or a large structural change of a protein which results in loss of function. Together with the fact that the mutations are strongly biased in males (14/14), *ZRSR2* most likely serves as a tumor suppressor gene via an X-linked recessive genetic mechanism of action. Remainding 9 *ZRSR2* mutations, which were missense mutations, were bound in both males (6 cases) and females (3 cases) while those originated from somatic cells were found only in two cases.

However, these missense nucleotides were not included in either the dbSNP database (Builds 131 and 132) or the 1000 human genome database (calling of snp as of May, 2011) . This suggests that many of these missenses SNV, if not all, likely represent functional somatic mutations found particularly in males. Although these mutation hot spots in *U2AF35* and *SRSF2* were investigated, mutations were not found in ALL (N= 24) or lymphoid tumors such as non-Hodgkin's lymphoma (N= 87).

All of the gene mutations are specific to MDS, and the mutation frequency in other diseases, especially in myeloid tumors is low. Some cases of AML include those which are progressed from MDS, but the mutation frequency in these gene clusters is significantly higher than that of new AML (de *novo* AML). This is also effective for distinguishing AML from MDS-derived AML.

Particularly in RARS, the mutation rate in the *SF3B1* gene is high, and these gene mutations are essentially determinants of this disease. It has been statistically shown that a group having a mutation in the *SRSF2* gene often shows a mutation in other genes. Frequent occurrence of *SRSF2* gene mutations is also a characteristic to CMML.

A disease group classified into RCMD according to the current WTO classification can be divided into two groups depending on the presence of *SF3B1, SRSF2* gene mutations. The prognosis of a group having these mutations may be better than that of a group having no mutation. Therefore, this is useful for prognostic prediction.

Spliceosome mutations in myeloid dysplasia widely affected the main components of the splicing complexes E/A in a mutually exclusive fashion. This logically suggested that common functional targets of these mutations are exact recognition of an exon-intron boundary, and subsequent recruitment of U2 snRNP to Pre-mRNA via these complexes (Nonpatent Literatures 1, 5).

In order to understand this and in order to obtain an insight about biological/biochemical effects resulted from these splicing mutations, the wild type *U2AF35* and the mutant (S34F) *U2AF35* were expressed in HeLa cells by the gene transfer method with retrovirus using EGFP as a marker.

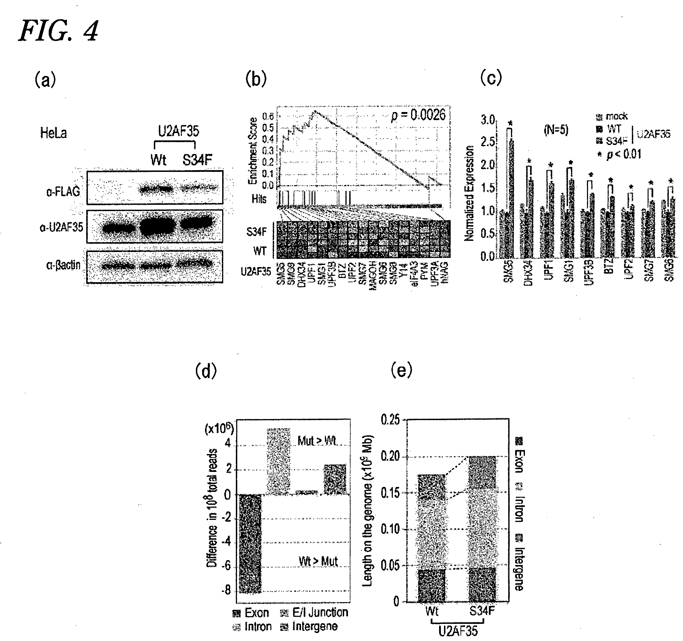

Fig. 4 (a) shows a photograph of western-blot-analysis. Shown is the expression of the wild type *U2AF35* or the mutant (S34F) *U2AF35* introduced in HeLa cells, TF-1 cells used for the gene expression analysis.

GFP positive cells were collected 48 hours after the gene transfer. Then gene expression profiles of the mutant *U2AF35* introduced cells and the wild type *U2AF35* introduced cells were analyzed using GeneChip® human genome U133 plus 2.0 array analysis and gene set enrichment analysis (GSEA). Subsequently gene set enrichment analysis (GSEA) was performed (Nonpatent Literature 6).

In the present GSEA, all of the expressed genes were first ranked in descending order of differences in gene expression between the wild type *U2AF35* introduced cells and the mutant *U2AF35* introduced cells. Next a gene set enriched therein was searched. The initial GSEA results revealed several gene sets enriched in the mutant *U2AF35* introduced cells. Among these, particularly interesting was a gene set involved in nuclear RNA export (P=0.012). It is because a series of genes (N= 6) involved in nonsense-mediated mRNA decay (NMD) were included in the gene set, all of which were involved in core enrichment.

Fig. 4 (b) shows a graph illustrating the activation of the NMD pathway resulted from a *U2AF35* mutant, and shows the results from the initial GSEA using a gene set of c5.bp.v2.5. symbols (the Gene Ontology). Significant enrichment of a nuclear RNA export gene set in the mutant *U2AF35* introduced HeLa cells is shown as compared with the wild type *U2AF35* introduced cells.

In the next step, NMD-related genes were selected from the nuclear RNA export gene set to obtain additional NMD-related genes, which gave a more comprehensive NMD pathway gene set. More significant enrichment of the NMD gene set in the mutant *U2AF35* introduced HeLa cell is shown. The significance of the gene set was experimentally determined by rearrangement of the 1,000-gene set.

Indeed, this enrichment became even more significant when GSEA was performed in which a more comprehensive NMD gene set was included (P = 0.0026) (Nonpatent Literature 7). Microarray expression data were confirmed by quantitative polymerase chain reaction (qPCR).

Fig. 4 (c) shows the results from qPCR. Microarray results of the expression of 9 genes which contribute to the core enrichment of the NMD gene set have been obtained. After normalizing against the mean expression (+ S. E.) in the wild type *U2AF35* introduced HeLa cells, the mean expression (+ S. E.) of the NMD genes in pseudo introduced HeLa cells, wild type *U2AF35* introduced HeLa cells and mutant *U2AF35* introduced HeLa cells were plotted. P values were determined by the Mann-Whitney U tests.

Similar enrichment was also observed in a gene expression profile of S34F mutant-introduced TF-1 (a myelodysplastic syndromes-derived cell line in which no spliceosome mutation has been known).

Results from the GSEA of *U2AF35* mutant-introduced TF-1 cells showed that the NMD pathway gene set was enriched in *U2AF35* introduced cells.

Given that the NMD pathway known as a mRNA quality control mechanism provides a post-transcription mechanism where an abnormal transcription product which prematurely terminates translation is recognized and eliminated (Nonpatent Literature 8), the GSEA results strongly suggested that an abnormal transcription which most likely produces a non-splicing RNA species takes place to induce an NMD activity in mutant *U2AF35* introduced cells.

In order to confirm this, a GeneChip® human exon 1.0 ST array (Affymetrix Inc.) was used to perform total transcriptome analysis of these cells. In the analysis, two discrete probe sets showing different levels of evidence that they are exons, i.e., the "core" set (true exon) and the "non-core" set (most likely intron) were individually followed for their behaviors. Results showed that the core set probes and the non-core set probes were differently enriched in probes showing significantly different expression between the wild type introduced cells and the mutant introduced cells (FDR = 0.01) . The core set probes were significantly enriched in probes significantly downregulated in the mutant *U2AF35* introduced cells as compared with wild type *U2AF35* introduced cells whereas the non-core set probes were significantly enriched in probes significantly upregulated in the mutant *U2AF35* introduced cells. Even when all of the probe sets were included, significantly different enrichments were shown.

Further, the core set probes which showed significant differential expression tended to be upregulated and downregurlated in the wild type *U2AF35* introduced cells and the mutant *U2AF35* introduced cells, respectively, as compared with the pseudo introduced cells, but the non-core set probes which showed differential expression also showed the same trend.

That is, these exon array results suggested that the wild type *U2AF35* correctly promoted true RNA splicing whereas the mutant *U2AF35* likely inhibited this process to produce a non-core, resulting in an unspliced intron sequence left behind.

In order to confirm the results from the exon array analysis and in order to show more direct evidence of abnormal splicing in mutant introduced cells, sequencing analysis was performed for mRNA extracted from HeLa cells in which expressions of the wild type *U2AF35* and the mutant (S34F) *U2AF35* were induced by doxycycline. Differential enrichment of the probe sets was reproduced between the two HeLa samples by directly counting respective readings of the core set probes and the non-core set probes.

Abnormal splicing in the mutant *U2AF35* introduced cells was more directly proved by the evaluation of reading counts at various segments, i.e., exons, introns and intergenic regions as well as exon/intron junction regions. First, after adjusted against the total mapped readings, the wild type *U2AF35* introduced cells showed increased readings in exon segments, but decreased readings in other segments as compared with the mutant *U2AF35* introduced cells. Readings from the mutant introduced cells were more broadly mapped across the genome region as compared with the wild type *U2AF35* introduced cells, which could be roughly explained by non-exon readings.

Fig. 4 (d) and (e) show results from the studies in which the wild type (normal) *U2AF35* and the mutant *U2AF35* were expressed in HeLa cells, and RNA sequencing was performed to compare the wild type with the mutant to investigate an expression ratio of exons and introns. Exons, introns and the like in the genomes of respective cells in which the wild type gene or the mutant gene was introduced are shown in Fig. 4 (e) .

In a case where the wild type gene was introduced, many exons but almost no introns were observed. In contrast, in a case where the mutant gene was introduced, many introns but almost no exons were observed.

Further, when true exon/intron junction regions were included, the number of readings was significantly increased in the mutant *U2AF35* introduced cells as compared with in the wild type *U2AF35* introduced cells. These results clearly showed that failure in splicing ubiquitously occurred in the mutant *U2AF35* introduced cells.

Next, effects of a functional disorder of the E/A splicing complex due to a gene mutation on a phenotype of a hematopoietic cell were studied. First, lentivirus constructs which express the S34F *U2AF35* mutant and the wild type *U2AF35* under the control of a tetracycline inducible promoter were introduced into TF-1 cells and HeLa cells. After inducing their expression, effects of the *U2AF35* mutant on cell proliferation were studied.

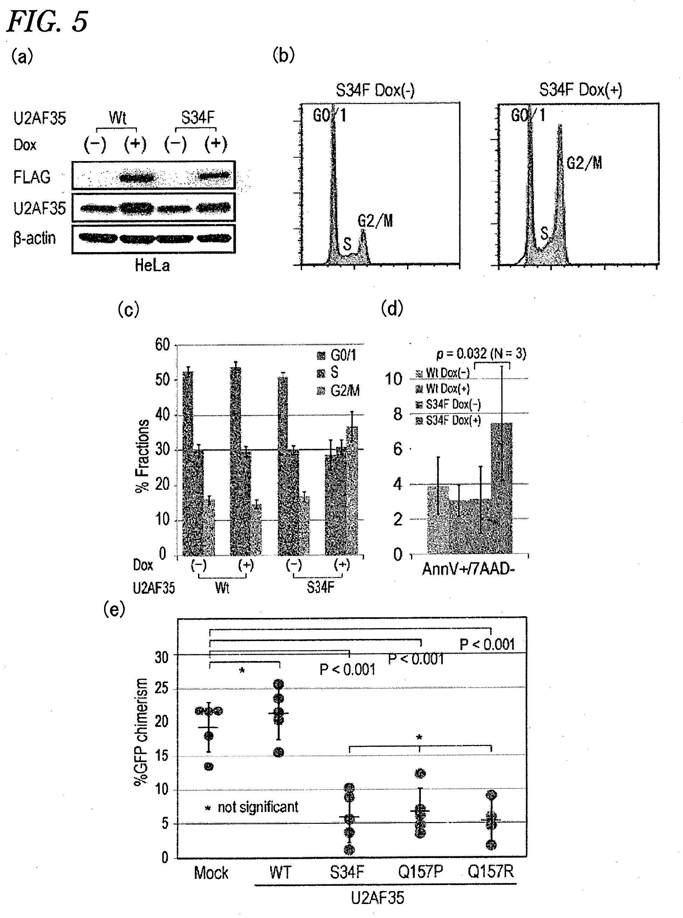

Fig. 5 (a) shows a photograph of western blot analysis. It shows doxycycline inducible expression of the mutant (S34F) and the wild type *U2AF35* in the HeLa cells and the TF-1 cells 72 hours after induction.

Unexpectedly, after inducing gene expression with doxycycline, the mutant *U2AF35* introduced cells showed decreased cell proliferation, which was not observed in the wild type *U2AF35* introduced cells.

When examining the functional analysis of the mutant *U2AF35,* the cell proliferation assays of the HeLa cells and the TF-1 cells showed that growth was significantly suppressed after inducing *U2AF35* expression in the mutant *U2AF35* introduced cells, but not in the wild type *U2AF35* introduced cells.

In order to confirm these observation results in primary cultured cells, either mutant (S34F, Q157P, Q157R) constructs or a wild type *U2AF35* construct and a pseudo construct each having an EGFP marker were introduced into a highly purified hematopoietic stem cell population (CD34⁻ c-Kit⁺ ScaI⁺ Lin⁻, CD34⁻KSL) prepared from the bone marrow of a C57BL/6 (B6)-Ly5.1 mouse.

Next, the bone marrow reconstitution ability of these gene transfer cells was studied by the competitive bone marrow reconstitution assay. After mixed with total bone marrow cells from a B6-Ly5.1/5.2 F1 mouse, the gene transfer cells were transplanted into a B6-Ly5.2 recipient which had received a lethal dose of radiation. Six weeks later, peripheral blood chimaerism originated from GFP positive cells was evaluated by flow cytometry.

Evaluation of EGFP% in the gene transfer cells and overall proliferation by *ex vivo* follow-up studies confirmed that each recipient mouse had received the similar number of GFP positive cells among various retrovirus groups.

Fig. 5 (b) and (c) show graphs illustrating how the wild type (normal) *U2AF35* gene expressed in HeLa cells affects cell cycles. When the mutated gene is expressed, many cells are found to be arrested at G2/M. Further, in a case where the wild type *U2AF35* gene is expressed, similar cell cycles are observed even when compared with the control group in which neither of the genes is expressed. This suggests that-the cell cycles are arrested at G2/M only in those where the mutated genes are expressed.

Fig. 5 (d) shows a graph illustrating that the expression of the wild type (normal) and the mutant *U2AF35* in HeLa cells has induced apoptosis. Induction of apoptosis is detected using positive anexyn V and negative 7AAD as indicators. Only the group in which the mutated gene is expressed is found to show apoptosis.

Fig. 5 (e) shows the results from the competitive bone marrow reconstitution assay of the CD34⁻KSL cells to which one of the 3 *U2AF35* mutants is introduced, as compared with the pseudo introduced cells and the wild type *U2AF35* introduced cells. A horizontal line and a vertical line show the mean and S.D. of the results obtained from 5 mice, respectively. Outliers were excluded from the analysis by the Grubbs' outlier test. The significance in differences was determined by the Bonferroni multiple comparison test. The vertical axis shows GFP positive Ly5.1 cell % in peripheral blood after 6 weeks of transplantation.

The wild type *U2AF35* introduced cells showed slightly higher bone marrow reconstruction ability than the pseudo introduced cells. In contrast, recipients of the cells to which one of the *U2AF35* mutants was introduced showed significantly lower GFP⁺ cell chimaerism than those received the pseudo introduced cells or the wild type *U2AF35* introduced cells, showing that the bone marrow reconstruction ability of hematopoietic stem/precursor cells in which a *U2AF35* mutant was expressed was impaired. The opposite behaviors of the wild type *U2AF35* construct and the mutant *U2AF35* construct also suggested that these mutants caused the loss of the *U2AF35* function probably through a dominant negative effect against the wild type protein.

The whole exome sequencing studies described above have revealed the complexity of the novel pathway mutations found in about 40 to 60% of myeloid dysplasia patients which affect multiple different components of the splicing machinery (the splicing complexes E/A).

The RNA splicing system is a unique characteristic of metazoan species. In this system, a coding sequence is prepared as multiple fragments which are separately located in the genome DNA. After transcription, intervening sequences are deleted to reconnect them, creating a functional mRNA copy (Nonpatent Literature 1). This appears to be a tedious or wasteful process. However, major protein resources are provided by this, and the functional diversity is provided by alternative splicing in spite of the limited number of the genes (Nonpatent Literature 5) .

Accordingly, if the integrity of the whole transcriptome is assured by sophisticatedly adjusting such a complicated process using the spliceosome machinery, we certainly have to pay a price in return for it. Abnormal RNA splicing, i.e., cancer specific alternative splicing is implicated in the development of human cancers including myelodysplastic syndromes and other hematopoietic tumors although the exact mechanism thereof has not been elucidated.

Considering that the present exome sequencing appeared to have a sufficient range and sensitivity for detecting other spliceosome components, it is safe to say that other spliceosome components were not affected although spliceosome mutations were broadly and specifically found in the components involved in the splicing complexes E/A. These mutations were almost completely mutually exclusive and very specific to myeloid dysplasia. This suggested that a functional disorder of these complexes is a common consequence of these mutations, and they are involved in the development of this distinct category of myeloid tumors by disturbing a physiological process of RNA splicing. Although there is no direct evidence to prove that these mutations actually create abnormally spliced mRNA species, the enhanced NMD activity in the mutant *U2AF35* introduced cells strongly suggested that the mutant *U2AF35* promotes increased production of mRNA species having a premature termination codon.

Meanwhile, the results from the competitive bone marrow reconstitution assay and the *in vitro* cell proliferation assay can not be easily interpreted. The mutant *U2AF35* appeared to suppress cell growth/proliferation instead of providing an advantage for growth and facilitating clonal selection as expected from classical oncogenes. Interestingly, with regard to the observation results, there is a report that apoptosis was induced by the *ZRSR2* knockdown in HeLa cells, which supports the common consequence of these pathway mutations (Nonpatent Literature 9). Although there is no clear answer for the apparently contradicting phenotype, this suggests that a pathogenetic role of a *U2AF35* mutation needs to be understood in the context of mutations in other genes and/or neoplastic growth of *U2AF35* mutant cells or a tumor cell environment compatible with clonal selection.

It should be noted that the most common clinical manifestation of myelodysplastic syndromes is not uncontrolled cell proliferation, but is serious cytopenia in multiple cell lines due to ineffective hematopoiesis accompanied by increased apoptosis. In this regard, the results from the studies about the development 5q-syndrome reporting that apoptosis of an erythrocyte precursor is increased due to *RPS14* haploinsufficiency, but myeloid proliferation is not increased can provide a suggestion.

The present discovery of highly frequent mutations in the spliceosome components clearly showed the importance of disordered RNA splicing in the development of myelodysplastic syndromes and related myeloid tumors. Many *ZRSR2* mutations cause early truncation of a protein while mutations in *U2AF35, SFRS2* and *SF3B1* appear to target specific amino acid positions, and appear to be gain-of-function types. Such gain-of-function can be explained by any dominant negative mechanism. These mutations appear to be involved in a common step of RNA splicing. However, there also exits a difference in their distribution among disease types. In CMML, the frequency of *SFRS2* mutations is high, which are also often overlapped with other common mutations.

Further, the present invention can provide a method of screening for a candidate therapeutic agent or a candidate prophylactic agent for myelodysplastic syndromes or a myelogenous tumor.

That is, the method comprises the steps of evaluating whether a test substance can inhibit an expression or an activity of a protein translated from at least one gene of the *U2AF35* gene, the *ZRSR2* gene, the *SFRS2* gene and the *SF3B1* gene using a sample containing human genes of a subject; and selecting the test substance capable of inhibiting the expression or the activity of the protein translated from said at least one gene as an effective substance for preventing or treating a state or a disease resulted from myelodysplastic syndromes or a myelogenous tumor.

The method may comprise the steps of administering a test substance to an non-human animal; measuring an expression or an activity of at least one gene of the *U2AF35* gene, the *ZRSR2* gene, the *SFRS2* gene and the *SF3B1* gene in the non-human animal to which the test substance is administered; and selecting the test substance capable of inhibiting the expression or the activity as an effective substance.

A mutated gene may be noted as a similar method of screening for a candidate therapeutic agent or a candidate prophylactic agent.

That is, the method comprises the steps of evaluating whether a test substance can inhibit an expression or an activity of a protein translated from at least one gene of the *U2AF35* gene, the *ZRSR2* gene, the *SFRS2* gene and the *SF3B1* gene using a sample containing human genes of a subject; and selecting the test substance capable of inhibiting the expression or the activity of the protein translated from said at least one gene as an effective substance for preventing or treating a state or a disease resulted from myelodysplastic syndromes or a myelogenous tumor.

The method may comprise the steps of administering a test substance to an non-human animal; determining a mutation in at least one gene of the *U2AF35* gene, the *ZRSR2* gene, the *SFRS2* gene and the *SF3B1* gene in the non-human animal to which the test substance is administered; and selecting the test substance capable of decreasing the mutation as an effective substance.

For the mutations in the above genes, a substitution of S with F or Y at an amino acid residue at position 34 of a protein translated from the *U2AF35* gene, substitution of Q with R or P at an amino acid residue at position 157 of the protein translated from the *U2AF35* gene, any inactivating mutation in a protein translated from the *ZRSR2* gene, a substitution of P with H or L or R at an amino acid residue at position 95 of a protein translated from the *SFRS2* gene, a substitution of K with E at an amino acid residue at position 700, a substitution of E with D at an amino acid residue at position 622, a substitution of H with Q or D at an amino acid residue at position 662, a substitution of K with N or T or E or R at an amino acid residue at position 666 of a protein translated from the *SF3B1* gene may be effectively used.

For the test substances, any known compounds and novel compounds may be used, including, for example, organic small molecule compounds, compound libraries created by the combinatorial chemistry technology, nucleic acids (nucleosides, oligonucleotides, polynucleotides and the like), carbohydrates (monosaccharides, disaccharides, oligosaccharides, polysaccharides and the like), lipids (saturated or unsaturated linear, branched, cyclic fatty acids and the like), amino acids, proteins (oligopeptides, polypeptides and the like), random peptide libraries created by solid phase synthesis and the phage display method, natural compounds from microorganisms, animals and plants, marine organisms and the like.

Non-human animals include, for example, mammals such as mouse, rat, hamster, guinea pig, rabbit, canine, monkey and the like.

In a case where a non-human animal is used, conventionally known methods can be used for administering a test substance to the non-human animal. For example, they include oral administration, parenteral administration (intravenous injection, subcutaneous injection, intraperitoneal injection, local infusion and the like). Dosage, dosing interval, dosing period and the like are to be suitably selected depending on the test substance and the animal to be used.

In order to evaluate the efficacy of a test substance, conventionally known methods may be used. For example, expression levels of proteins translated from the above genes or mutated genes thereof in a non-human animal to which the test substance is administered may be measured.

The expression levels can be measured by, for example, obtaining a biological sample such as bone marrow and blood from a non-human animal, and measuring a transcription product and the like in the sample.

Further, screening may be performed using tissues and cells, and biological materials, for example, bone marrow, blood and the like from a non-human animal.

Cells in which expression levels of proteins translated from the above genes or mutated genes thereof can be directly evaluated are their expression cells while cells in which the expression levels can be indirectly evaluated are those in which reporter assays can be performed for the transcriptional regulatory domains of the above genes or mutated genes thereof.

Cells in which reporter assays can be performed for the transcriptional regulatory domains of the above genes or mutated genes thereof are those having the transcriptional regulatory domains of the above genes or mutated genes thereof, reporter genes operationally linked to the domains.

Further, cells having the above genes or mutated genes thereof or cells in which mutations are forced to be expressed can be used to evaluate whether a subject have predisposition for another disease different from myelodysplastic syndromes or a myelogenous tumor.

### INDUSTRIAL APPLICABILITY

The present invention is effective for early treatment and prevention of myelodysplastic syndromes or a myelogenous tumor, and industrially useful.

### mRNA AND AMINO ACID SEQUENCES:

The nucleotide sequences of the wild type human mRNAs for the U2AF35 gene, the ZRSR2 gene, the SRSF2 gene and the SF3B1 gene, and the corresponding amino acid sequences, to which reference is made herein in the claims, are presented in the attached sequence listing. The listing contains the following sequences:
SEQ ID No: 1:
   Wild type U2AF35 mRNA (according to accession number NM_006758.2 of 16/07/2011) together with the amino acid sequence translated from the coding sequence (nt 85..807).
SEQ ID No: 2:
   Amino acid sequence of the wildtype U2AF35 protein translated from the coding sequence (nt 85..807) of the mRNA according to accession number NM_006758.2 of 16/07/2011 (see SEQ ID NO: 1) .
SEQ ID No: 3:
   Wild type ZRSR2 mRNA (according to accession number NM_005089.3 of 14.02.2011) together with the amino acid sequence translated from the coding sequence (nt 46..1494).
SEQ ID No: 4:
   Amino acid sequence of the wildtype ZRSR2 protein translated from the coding sequence (nt 46..1494) of the mRNA according to accession number NM_005089.3 of 14.02.2011 (see SEQ ID NO: 3) .
SEQ ID No: 5:
   Wild type SRSF2 mRNA (according to accession number NM_003016.4 of 30/07/2011) together with the amino acid sequence translated from the coding sequence (nt 252..917).
SEQ ID No: 6:
   Amino acid sequence of the wildtype SRSF2 protein translated from the coding sequence (nt 252..917) of the mRNA according to accession number NM_003016.4 of 30/07/2011 (see SEQ ID NO: 5) .
SEQ ID No: 7:
   Wild type SF3Bl mRNA together with the amino acid sequence translated from the coding sequence (nt 49..3963), sequence as known at the priority date
SEQ ID No: 8:
   Amino acid sequence of the wildtype SF3Bl protein translated from the coding sequence (nt 49..3963) of the mRNA presented in SEQ ID NO: 7).

## Claims

1. A method of evaluating whether a subject has myelodysplastic syndromes or a myelogenous tumor, the method comprising the step of
detecting a gene mutation in at least one of the *U2AF35* gene, the *ZRSR2* gene, the *SRSF2* gene and the *SF3B1* gene using a sample containing human genes of the subject wherein the subject is evaluated to have myelodysplastic syndromes or a myelogenous tumor in a case where at least one of the following is detected:
a mutation in the *U2AF35* gene resulting in a substitution of S with F or Y at an amino acid residue at position 34 of a protein translated from the *U2AF35* gene, the amino acid sequence of the protein being compared to the amino acid sequence of a protein translated from the mRNA having the accession number NM_006758.2 (SEQ ID NO: 1),
a mutation in the *U2AF35* gene resulting in a substitution of Q with R or P at an amino acid residue at position 157 of the protein translated from the *U2AF35* gene, the amino acid sequence of the protein being compared to the amino acid sequence of a protein translated from the mRNA having the accession number NM_006758.2 (SEQ ID NO: 1),
a mutation in the *ZRSR2* gene resulting in a protein translated from the *ZRSR2* gene, the protein having a substitution of I with T at position 53, a substitution of E with G at position 133, a substitution of I with N at position 202, a substitution of F with V at position 239, a substitution of N with Y at position 261, a substitution of C with R at position 302, a substitution of C with R at position 326, a substitution of H with R at position 330, or a substitution of N with K at position 382, the amino acid sequence of the protein being compared to the amino acid sequence of a protein translated from the mRNA having the accession number NM_005089.3 (SEQ ID NO: 3),
a nonsense mutation in the *ZRSR2* gene resulting in a mutation of a codon coding for S at position 40 in the wild type protein to a stop codon, a mutation of a codon coding for R at position 126 in the wild type protein to a stop codon, a mutation of a codon coding for E at position 148 in the wild type protein to a stop codon, a mutation of a codon coding for W at position 291 in the wild type protein to a stop codon, a mutation of a codon coding for Y at position 347 in the wild type protein to a stop codon, or a mutation of a codon coding for E at position 362 in the wild type protein to a stop codon, wherein the amino acid sequence of the wild type protein is the amino acid sequence of the protein translated from the mRNA having the accession number NM_005089.3 (SEQ ID NO: 3),
a frameshift mutation in the *ZRSR2* gene resulting in a frameshift at position 96 of the wild type protein, a frameshift at position 118 of the wild type protein, a frameshift at position 237 of the wild type protein, a frameshift mutation at position 323 of the wild type protein, or a frameshift at position 327, wherein the amino acid sequence of the wild type protein is the amino acid sequence of the protein translated from the mRNA having the accession number NM_005089.3 (SEQ ID NO: 3),
a splice-site mutation in the *ZRSR2* gene of R at position 68, or a splice-site mutation of K at position 257, wherein the amino acid sequence of the wild type protein is the amino acid sequence of the protein translated from the mRNA having the accession number NM_005089.3 (SEQ ID NO: 3),
a mutation in the *SRSF2* gene resulting in a substitution of P with H or L or R at an amino acid residue at position 95 of a protein translated from the *SRSF2* gene, the amino acid sequence of the protein being compared to the amino acid sequence of a protein translated from the mRNA having the accession number NM_003016.4,
a mutation in the *SF3B1* gene resulting in a substitution of K with E at an amino acid residue at position 700, a substitution of E with D at an amino acid residue at position 622, a substitution of H with Q or D at an amino acid residue at position 662, or a substitution of K with N or T or E or R at an amino acid residue at position 666 of a protein translated from the *SF3B1* gene, the amino acid sequence of the protein being compared to the amino acid sequence of the wild type *SF3B1* protein (SEQ ID NO: 8).

2. Use of a polypeptide comprising at least a portion of the gene product of a mutated *U2AF35* gene, the polypeptide having at least one of a substitution of S with F or Y at an amino acid residue at position 34 or a substitution of Q with R or P at an amino acid residue at position 157, the amino acid sequence of the polypeptide being compared to the amino acid sequence of the protein translated from the mRNA having the accession number NM_006758.2 (SEQ ID NO: 1), as a marker for evaluating whether a subject has myelodysplastic syndromes or a myelogenous tumor.

3. Use of a polypeptide comprising at least a portion of the gene product of a mutated *ZRSR2* gene, the polypeptide having at least one of a substitution of I with T at position 53, a substitution of E with G at position 133, a substitution of I with N at position 202, a substitution of F with V at position 239, a substitution of N with Y at position 261, a substitution of C with R at position 302, a substitution of C with R at position 326, a substitution of H with R at position 330, or a substitution of N with K at position 382, the amino acid sequence of the polypeptide being compared to the amino acid sequence of the protein translated from the mRNA having the accession number NM_005089.3 (SEQ ID NO: 3),
as a marker for evaluating whether a subject has myelodysplastic syndromes or a myelogenous tumor.

4. Use of a polypeptide comprising at least a portion of the gene product of a mutated *SRSF2* gene, the polypeptide having at least one of a substitution of P with H or L or R at an amino acid residue at position 95, the amino acid sequence of the polypeptide being compared to the amino acid sequence of a polypeptide translated from the mRNA having the accession number NM_003016.4 (SEQ ID NO: 5), as a marker for evaluating whether a subject has myelodysplastic syndromes or a myelogenous tumor.

5. Use of a polypeptide comprising at least a portion of the gene product of a mutated *SF3B1* gene, the polypeptide having at least one of a substitution of K with E at an amino acid residue at position 700, a substitution of E with D at an amino acid residue at position 622, a substitution of H with Q or D at an amino acid residue at position 662, or a substitution of K with N or T or E or R at an amino acid residue at position 666, the amino acid sequence of the polypeptide being compared to the amino acid sequence of the wild type *SF3B1* protein (SEQ ID NO: 8), as a marker for evaluating whether a subject has myelodysplastic syndromes or a myelogenous tumor.

## Patentansprüche

1. Verfahren zum Bewerten, ob eine Person myelodysplastische Syndrome oder einen myelogenen Tumor hat, wobei das Verfahren den Schritt umfasst des:
Detektierens einer Genmutation in mindestens einem der folgenden Gene: dem *U2AF35-Gen,* dem ZRSR2-Gen, dem *SRSF2-Gen* und dem *SF3B1*-Gen, unter Verwendung einer Probe, die menschliche Gene der Person enthält, wobei die Person so bewertet wird, dass sie myelodysplastische Syndrome oder einen myelogenen Tumor hat, wenn mindestens eines von Folgendem detektiert wird:
eine Mutation im *U2AF35*-Gen, die zu einer Substitution von S mit F oder Y an einem Aminosäurerest an Position 34 eines von dem *U2AF35-Gen* translatierten Proteins führt, wobei die Aminosäuresequenz des Proteins mit der Aminosäuresequenz eines von der mRNA mit der Zugriffsnummer NM_006758.2 (SEQ ID NO: 1) translatierten Proteins verglichen wird,
eine Mutation im *U2AF35-Gen,* die zu einer Substitution von Q mit R oder P an einem Aminosäurerest an Position 157 des vom *U2AF35-Gen* translatierten Proteins führt, wobei die Aminosäuresequenz des Proteins mit der Aminosäuresequenz eines von der mRNA mit der Zugriffsnummer NM_006758.2 (SEQ ID NO: 1) translatierten Proteins verglichen wird,
eine Mutation im *ZRSR2-Gen,* die zu einem von dem *ZRSR2*-Gen translatierten Protein führt, wobei das Protein eine Substitution von I mit T an Position 53, eine Substitution von E mit G an Position 133, eine Substitution von I mit N an Position 202, eine Substitution von F mit V an Position 239, eine Substitution von N mit Y an Position 261, eine Substitution von C mit R an Position 302, eine Substitution von C mit R an Position 326, eine Substitution von H mit R an Position 330, oder eine Substitution von N mit K an Position 382 aufweist, wobei die Aminosäuresequenz des Proteins mit der Aminosäuresequenz eines von der mRNA mit der Zugriffsnummer NM_005089.3 (SEQ ID NO: 3) translatierten Proteins verglichen wird,
eine Nonsense-Mutation im *ZRSR2-Gen,* die zu einer Mutation eines im Wildtyp-Protein an Position 40 für S kodierenden Codons zu einem Stop-Codon, einer Mutation eines im Wildtyp-Protein an Position 126 für R kodierenden Codons zu einem Stop-Codon, einer Mutation eines im Wildtyp-Protein an Position 148 für E kodierenden Codons zu einem Stop-Codon, einer Mutation eines im Wildtyp-Protein an Position 291 für W kodierenden Codons zu einem Stop-Codon, einer Mutation eines im Wildtyp-Protein an Position 347 für Y kodierenden Codons zu einem Stop-Codon, oder einer Mutation eines im Wildtyp-Protein an Position 362 für E kodierenden Codons zu einem Stop-Codon führt, wobei die Aminosäuresequenz des Wildtyp-Proteins die Aminosäuresequenz des von der mRNA mit der Zugriffsnummer NM_005089.3 (SEQ ID NO: 3) translatierten Proteins ist,
eine Frameshift-Mutation im *ZRSR2-Gen,* die zu einem Frameshift an Position 96 des Wildtyp-Proteins, einem Frameshift an Position 118 des Wildtyp-Proteins, einem Frameshift an Position 237 des Wildtyp-Proteins, einer Frameshift-Mutation an Position 323 des Wildtyp-Proteins, oder einem Frameshift an Position 327 führt, wobei die Aminosäuresequenz des Wildtyp-Proteins die Aminosäuresequenz des von der mRNA mit der Zugriffsnummer NM_005089.3 (SEQ ID NO: 3) translatierten Proteins ist,
eine Splicestellen-Mutation im *ZRSR2-Gen* von R an Position 68, oder eine Splicestellen-Mutation von K an Position 257, wobei die Aminosäuresequenz des Wildtyp-Proteins die Aminosäuresequenz des von der mRNA mit der Zugriffsnummer NM_005089.3 (SEQ ID NO: 3) translatierten Proteins ist,
eine Mutation des *SRSF2*-Gens*,* die zu einer Substitution von P mit H oder L oder R an einem Aminosäurerest an Position 95 eines vom SRSF2-Gen translatierten Proteins führt, wobei die Aminosäuresequenz des Proteins mit der Aminosäuresequenz eines von der mRNA mit der Zugriffsnummer NM_003016. 4 translatierten Proteins verglichen wird,
eine Mutation im *SF3B1*-Gen*,* die zu einer Substitution von K mit E an einem Aminosäurerest an Position 700, einer Substitution von E mit D an einem Aminosäurerest an Position 622, einer Substitution von H mit Q oder D an einem Aminosäurerest an Position 662, oder einer Substitution von K mit N oder T oder E oder R an einem Aminosäurerest an Position 666 eines vom *SF3B1*-Gen translatierten Proteins führt, wobei die Aminosäuresequenz des Proteins mit der Aminosäuresequenz des Wildtyp-*SF3B1*-Proteins (SEQ ID NO: 8) verglichen wird,

2. Verwendung eines Polypeptids umfassend mindestens einen Teil des Genproduktes eines mutierten U2AF35-Gens, wobei das Polypeptid mindestens eine Substitution von S mit F oder Y an einem Aminosäurerest an Position 34 oder eine Substitution von Q mit R oder P an einem Aminosäurerest an Position 157 aufweist, wobei die Aminosäuresequenz des Polypeptids mit der Aminosäuresequenz des von der mRNA mit der Zugriffsnummer NM_006758.2 (SEQ ID NO: 1) translatierten Proteins verglichen wird, als Marker zur Bewertung, ob eine Person myelodysplastische Syndrome oder einen myelogenen Tumor hat.

3. Verwendung eines Polypeptids umfassend mindestens einen Teil des Genproduktes eines mutierten ZRSR2-Gens, wobei das Polypeptid mindestens eine Substitution von I mit T an Position 53, eine Substitution von E mit G an Position 133, eine Substitution von I mit N an Position 202, eine Substitution von F mit V an Position 239, eine Substitution von N mit Y an Position 261, eine Substitution von C mit R an Position 302, eine Substitution von C mit R an Position 326, eine Substitution von H mit R an Position 330, oder eine Substitution von N mit K an Position 382 aufweist, wobei die Aminosäuresequenz des Polypeptids mit der Aminosäuresequenz des von der mRNA mit der Zugriffsnummer NM_005089.3 (SEQ ID NO: 3) translatierten Proteins verglichen wird,
als Marker zur Bewertung, ob eine Person myelodysplastische Syndrome oder einen myelogenen Tumor hat.

4. Verwendung eines Polypeptids umfassend mindestens einen Teil des Genproduktes eines mutierten SRSF2-Gens, wobei das Polypeptid mindestens eine Substitution von P mit H oder L oder R an einem Aminosäurerest an Position 95 aufweist, wobei die Aminosäuresequenz des Polypeptids mit der Aminosäuresequenz des von der mRNA mit der Zugriffsnummer NM_003016.4 (SEQ ID NO: 5) translatierten Proteins verglichen wird, als Marker zur Bewertung, ob eine Person myelodysplastische Syndrome oder einen myelogenen Tumor hat.

5. Verwendung eines Polypeptids umfassend mindestens einen Teil des Genproduktes eines mutierten *SF3B1*-Gens*,* wobei das Polypeptid mindestens eine Substitution von K mit E an einem Aminosäurerest an Position 700, eine Substitution von E mit D an einem Aminosäurerest an Position 622, eine Substitution von H mit Q oder D an einem Aminosäurerest an Position 662, oder eine Substitution von K mit N oder T oder E oder R an einem Aminosäurerest an Position 666 aufweist, wobei die Aminosäuresequenz des Polypeptids mit der Aminosäuresequenz des Wildtyp-*SF3B1*-Proteins (SEQ ID NO: 8) verglichen wird, als Marker zur Bewertung, ob eine Person myelodysplastische Syndrome oder einen myelogenen Tumor hat.

## Revendications

1. Procédé d'évaluation du fait qu'un sujet souffre de syndromes myélodysplasiques ou d'une tumeur myéloïde, le procédé comprenant l'étape suivante
détection d'une mutation génique dans au moins l'un du gène *U2AF35,* du gène *ZRSR2,* du gène *SRSF2* et du gène *SF3B1* en utilisant un échantillon contenant les gènes humains du sujet dans lequel le sujet est évalué comme souffrant de syndromes myélodysplasiques ou d'une tumeur myéloïde dans un cas où au moins l'un de ce qui suit est détecté:
une mutation dans le gène *U2AF35* résultant en une substitution de S par F ou Y au niveau d'un résidu d'acide aminé à la position 34 d'une protéine traduite du gène *U2AF35,* la séquence d'acides aminés de la protéine étant comparée à la séquence d'acides aminés d'une protéine traduite de l'ARNm ayant le numéro d'accès NM_006758.2 (SEQ ID NO: 1),
une mutation dans le gène *U2AF35* résultant en une substitution de Q par R ou P au niveau d'un résidu d'acide aminé à la position 157 d'une protéine traduite du gène *U2AF35,* la séquence d'acides aminés de la protéine étant comparée à la séquence d'acides aminés d'une protéine traduite de l'ARNm ayant le numéro d'accès NM_006758.2 (SEQ ID NO: 1),
une mutation dans le gène *ZRSR2* résultant en une protéine traduite du gène *ZRSR2,* la protéine ayant une substitution d'I par T à la position 53, une substitution d'E par G à la position 133, une substitution d'I par N à la position 202, une substitution de F par V à la position 239, une substitution de N par Y à la position 261, une substitution de C par R à la position 302, une substitution de C par R à la position 326, une substitution de H par R à la position 330, ou une substitution de N par K à la position 382, la séquence d'acides aminés de la protéine étant comparée à la séquence d'acides aminés d'une protéine traduite de l'ARNm ayant le numéro d'accès NM_005089.3 (SEQ ID NO: 3),
une mutation non-sens dans le gène *ZRSR2* résultant en une mutation d'un codon codant pour S à la position 40 dans la protéine de type sauvage pour un codon stop, une mutation d'un codon codant pour R à la position 126 dans la protéine de type sauvage pour un codon stop, une mutation d'un codon codant pour E à la position 148 dans la protéine de type sauvage pour un codon stop, une mutation d'un codon codant pour W à la position 291 dans la protéine de type sauvage pour un codon stop, une mutation d'un codon codant pour Y à la position 347 dans la protéine de type sauvage pour un codon stop, ou une mutation d'un codon codant pour E à la position 362 dans la protéine de type sauvage pour un codon stop, dans lequel la séquence d'acides aminés de la protéine de type sauvage est la séquence d'acides aminés de la protéine traduite de l'ARNm ayant le numéro d'accès NM_005089.3 (SEQ ID NO: 3),
une mutation de décalage du cadre de lecture dans le gène *ZRSR2* résultant en un décalage de cadre de lecture à la position 96 de la protéine de type sauvage, un décalage du cadre de lecture à la position 118 de la protéine de type sauvage, un décalage du cadre de lecture à la position 237 de la protéine de type sauvage, un mutation de décalage du cadre de lecture à la position 323 de la protéine de type sauvage, ou un décalage de cadre de lecture à la position 327, dans lequel la séquence d'acides aminés de la protéine de type sauvage est la séquence d'acides aminés de la protéine traduite de l'ARNm ayant le numéro d'accès NM_005089.3 (SEQ ID NO : 3),
une mutation du site d'épissage dans le gène *ZRSR2* de R à la position 68, ou une mutation du site d'épissage de K à la position 257, dans lequel la séquence d'acides aminés de la protéine de type sauvage est la séquence d'acides aminés de la protéine traduite de l'ARNm ayant le numéro d'accès NM_005089.3 (SEQ ID NO: 3),
une mutation dans le gène *SRSF2* résultant en une substitution de P par H ou L ou R au niveau d'un résidu d'acide aminé à la position 95 d'une protéine traduite du gène *SRSF2,* la séquence d'acides aminés de la protéine étant comparée à la séquence d'acides aminés d'une protéine traduite de l'ARNm ayant le numéro d'accès NM_003016.4,
une mutation dans le gène *SF3B1* résultant en une substitution de K par E au niveau d'un résidu d'acide aminé à la position 700, une substitution de E par D au niveau d'un résidu d'acide aminé à la position 622, une substitution de H par Q ou D au niveau d'un résidu d'acide aminé à la position 662, ou une substitution de K par N ou T ou E ou R au niveau d'un résidu d'acide aminé à la position 666 d'une protéine traduite à partir du gène *SF3B1,* la séquence d'acides aminés de la protéine étant comparée à la séquence d'acides aminés de la protéine *SF3B1* de type sauvage (SEQ ID NO: 8).

2. Utilisation d'un polypeptide comprenant au moins une partie du produit génique d'un gène *U2AF35* muté, le polypeptide ayant au moins l'une d'une substitution de S par F ou Y au niveau d'un résidu d'acide aminé à la position 34 ou d'une substitution de Q par R ou P au niveau d'un résidu d'acide aminé à la position 157, la séquence d'acides aminés du polypeptide étant comparée à la séquence d'acides aminés de la protéine traduite de l'ARNm ayant le numéro d'accès NM_006758.2 (SEQ ID NO: 1), en tant que marqueur pour l'évaluation du fait qu'un sujet souffre de syndromes myélodysplasiques ou d'une tumeur myéloïde.

3. Utilisation d'un polypeptide comprenant au moins une partie du produit génique d'un gène *ZRSR2* muté, le polypeptide ayant au moins l'une d'une substitution d'I par T à la position 53, d'une substitution d'E par G à la position 133, d'une substitution d'I par N à la position 202, d'une substitution de F par V à la position 239, d'une substitution de N par Y à la position 261, d'une substitution de C par R à la position 302, d'une substitution de C par R à la position 326, d'une substitution de H par R à la position 330, ou d'une substitution de N par K à la position 382, la séquence d'acides aminés du polypeptide étant comparée à la séquence d'acides aminés de la protéine traduite de l'ARNm ayant le numéro d'accès NM_005089.3 (SEQ ID NO: 3),
en tant que marqueur pour l'évaluation du fait qu'un sujet souffre de syndromes myélodysplasiques ou d'une tumeur myéloïde.

4. Utilisation d'un polypeptide comprenant au moins une partie du produit génique d'un gène *SRSF2* muté, le polypeptide ayant au moins l'une d'une substitution de P par H ou L ou R au niveau d'un résidu d'acide aminé à la position 95, la séquence d'acides aminés du polypeptide étant comparée à la séquence d'acides aminés d'un polypeptide traduit de l'ARNm ayant le numéro d'accès NM_003016.4 (SEQ ID NO: 5), en tant que marqueur pour l'évaluation du fait qu'un sujet souffre de syndromes myélodysplasiques ou d'une tumeur myéloïde.

5. Utilisation d'un polypeptide comprenant au moins une partie du produit génique d'un gène *SF3B1* muté, le polypeptide ayant au moins l'une d'une substitution de K par E au niveau d'un résidu d'acide aminé à la position 700, d'une substitution d'E par D au niveau d'un résidu d'acide aminé à la position 622, d'une substitution de H par Q ou D au niveau d'un résidu d'acide aminé à la position 662, ou d'une substitution de K par N ou T ou E ou R au niveau d'un résidu d'acide aminé à la position 666, la séquence d'acides aminés du polypeptide étant comparée à la séquence d'acides aminés de la protéine SF3B1 de type sauvage (SEQ ID NO: 8), en tant que marqueur pour l'évaluation du fait qu'un sujet souffre de syndromes myélodysplasiques ou d'une tumeur myéloïde.
